(19) Europäisches Patentamt
European Patent Office
Office européen des brevets

(11) Veröffentlichungsnummer: **0 001 981**
**B1**

(12) # EUROPÄISCHE PATENTSCHRIFT

(45) Veröffentlichungstag der Patentschrift: **01.04.81**

(51) Int. Cl.³: **C 07 H 17/08**, A 61 K 31/70

(21) Anmeldenummer: **78101160.6**

(22) Anmeldetag: **14.10.78**

(54) **Neue 9-(Omega-heteroarylamino-alkylamino)-erythromycine, ihre Salze, Verfahren zu ihrer Herstellung und diese Verbindungen enthaltende Arzneimittel.**

(30) Priorität: **10.11.77 DE 2750288**

(43) Veröffentlichungstag der Anmeldung: **30.05.79 Patentblatt 79/11**

(45) Bekanntmachung des Hinweises auf die Patenterteilung: **01.04.81 Patentblatt 81/13**

(84) Benannte Vertragsstaaten: **BE CH DE FR GB LU NL SE**

(56) Entgegenhaltungen:
**DE-A-2 515 075**
**DE-A-2 515 077**
**FR-A-2 306 704**

(73) Patentinhaber: **Dr. Karl Thomae GmbH, Postfach 720, *D-7950 Biberach (Riss) (DE)***

(72) Erfinder: **Woitun, Eberhard, Dr. Dipl.-Chem., Stecherweg 17, D-7950 Biberach 1 (DE)**
Erfinder: **Wetzel, Bernd, Dr. Dipl.-Chem., Kapellenweg 21, D-7950 Biberach 1 (DE)**
Erfinder: **Maier, Roland, Dr. Dipl.-Chem., Eichendorffweg 36, D-7950 Biberach 1 (DE)**
Erfinder: **Reuter, Wolfgang, Dr. Dipl.-Chem., D-7951 Laupertshausen 139 (DE)**
Erfinder: **Lechner, Uwe, Dr., Panoramastrasse 12, D-7951 Ummendorf (DE)**
Erfinder: **Werner, Rolf G., Dr., Blumenstrasse 19, D-7950 Biberach 18 (DE)**
Erfinder: **Goeth, Hans, Dr., Oberer Bühl 6, D-7950 Biberach 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).



ACTORUM AG.

# Neue 9-(ω-Heteroarylamino-alkylamino)-erythromycine, ihre Salze, Verfahren zu ihrer Herstellung und diese Verbindungen enthaltende Arzneimittel

Die Erfindung betrifft neue 9-(ω-Heteroarylamino-alkylamino)-erythromycine der allgemeinen Formel I,

$R{-}NH{-}(CH_2)_n{-}NH{-}E$ (I)

deren pharmakologisch verträgliche Säureadditionssalze mit anorganischen oder organischen Säuren, Verfahren zur Herstellung dieser Verbindungen und diese Verbindungen enthaltende Arzneimittel.

In der obigen allgemeinen Formel I bedeuten:
E die Erythromycylgruppe, +

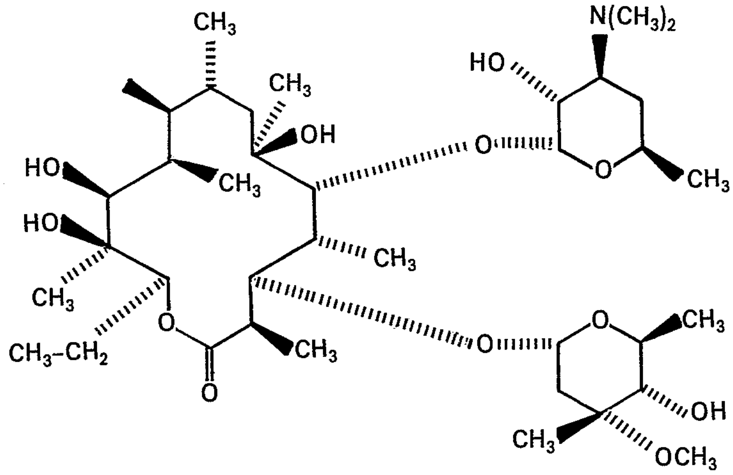

+ (Strukturformel nach E.H. Massey et al., J. Med. Chem. 17, 105 [1974])
n die Zahl 2 oder 3 und
R die 2-Pyrimidinylgruppe der allgemeinen Formel

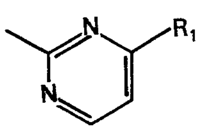

in der
$R_1$ ein Wasserstoffatom, eine Dialkylaminogruppe mit 2 bis 6 Kohlenstoffatomen, eine Phenoxyalkylaminogruppe mit 1–3 C-Atomen im Alkylenteil oder eine Phenylalkylaminogruppe mit 1 bis 3 Kohlenstoffatomen im Alkylenrest darstellt,
oder
R die 4-Pyrimidinylgruppe der allgemeinen Formel,

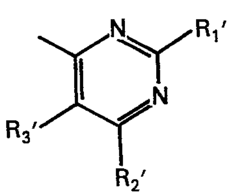

in der $R_1'$ ein Wasserstoffatom, die freie Aminogruppe, oder eine Dialkylaminogruppe mit 2 bis 6 Kohlenstoffatomen, darstellt,
$R_2'$ ein Wasserstoffatom, einen geradkettigen oder verzweigten Alkylrest mit 1 bis 3 Kohlenstoffatomen, einen Phenylrest, oder eine Dialkylaminogruppe mit insgesamt 2 bis 6 Kohlenstoffatomen und
$R_3'$ ein Wasserstoffatom, einen geradkettigen oder verzweigten Alkylrest mit 1 bis 3 Kohlenstoffatomen oder einen Phenylrest darstellt, oder
R die 2-Chinazolinylgruppe der allgemeinen Formel

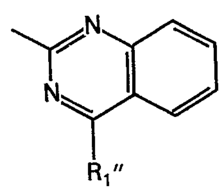

die 4-(5,6,7,8-Tetrahydro)-chinazolinylgruppe der allgemeinen Formel

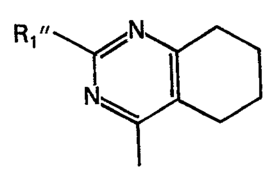

oder
die 2-Thieno[3,2-d]pyrimidinylgruppe der allgemeinen Formel

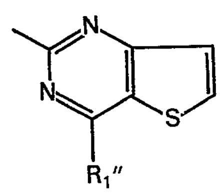

bedeutet

wobei in den obigen Formeln $R_1''$ ein Wasserstoffatom oder eine Dialkylaminogruppe mit 2 bis 6 Kohlenstoffatomen darstellt.

Sämtliche Verbindungen der allgemeinen Formel I und ihre Salze sind pharmakologisch wertvoll, sie sind insbesondere stark antibakteriell wirksam und bezüglich ihrer Wirkungen gegenüber grampositiven und gramnegativen Keimen und ihrer oralen Resorbierbarkeit den Verbindungen der FR-A-2 306 704 deutlich überlegen.

Die Verbindungen der allgemeinen Formel I können wie folgt hergestellt werden:

a) Durch Umsetzung eines Aminoalkylaminoerythromycins der allgemeinen Formel II,

$$H_2N-(CH_2)_n-NH-E \qquad (II)$$

in der n und E wie oben definiert sind, mit einer Verbindung der allgemeinen Formel III,

$$R-Z \qquad (III)$$

in der R wie oben definiert ist und Z eine leaving group, beispielsweise ein Halogenatom, eine Alkylsulfoxyl- oder Alkylsulfonylgruppe oder eine Alkylmercaptogruppe bedeutet, in einem polaren Lösungsmittel bei Temperaturen zwischen 20 und 150 °C.

Als Lösungsmittel dienen beispielsweise Alkohole, Dimethylformamid, Dimethylsulfoxid, Acetonitril, Dioxan, 1-Methyl-2-pyrrolidinon oder Hexamethylphosphorsäuretriamid. Ist Z ein Halogenatom, so führt man die Umsetzung vorzugsweise in Gegenwart eines halogenwasserstoffbindenden Mittels durch. Als halogenwasserstoffbindendes Mittel dient beispielsweise eine molare Menge einer anorganischen oder tertiären organischen Base, wie Natriumcarbonat oder Triäthylamin.

Die Verbindungen der allgemeinen Formel I können gegebenenfalls nachträglich in ihre physiologisch verträglichen Säureadditionssalze mit anorganischen oder organischen Säuren übergeführt werden. Als Säuren kommen beispielsweise Salzsäure, Bromwasserstoffsäure, Schwefelsäure, Essigsäure, Zitronensäure, Laurylsulfonsäure, Äpfelsäure in Betracht.

Die als Ausgangsstoffe dienenden Aminoalkylamino-erythromycine der allgemeinen Formel II können für den Fall, dass n die Zahl 3 bedeutet, durch Anlagerung von Acrylnitril an das Erythromycylamin (vgl. hierzu auch R. Ryden et al., J. Med. Chem. 16, 1059–1060 [1973]) und anschliessende Reduktion des sich gebildeten 9-N-(2-Cyanäthyl)-erythromycylamins mittels Wasserstoff in Gegenwart von feinverteilten Metallen wie Palladium, Platin, Raney-Nickel oder Raney-Kobalt in einem Lösungsmittel (Alkohole, Wasser oder cyclische Äther) gewonnen werden.

Soll eine Verbindung der Formel II hergestellt werden, in der n die Zahl 2 ist, so wird Nitroäthylen unter ähnlichen Bedingungen an das Erythromycylamin angelagert. Das dabei erhaltene 9-N-(2-Nitroäthyl)-erythromycylamin wird genauso, wie das oben beschriebene 9-N-(2-Cyanäthyl)-erythromycylamin, zu dem entsprechenden Aminoalkylaminoerythromycin der Formel II reduziert.

Die Ausgangsverbindungen der allgemeinen Formel III sind literaturbekannt oder lassen sich in Anlehnung an literaturbekannte Verfahrensweisen herstellen.

Das Erythromycylamin lässt sich durch katalytische Hydrierung von Erythromycin-oxim herstellen (vgl. E.H. Massey et al., J. Med. Chem. 17, 105–107 [1974]).

Die Verbindungen der allgemeinen Formel I besitzen wertvolle pharmakologische Eigenschaften, sie sind insbesonders wirksam gegen grampositive und gramnegative Bakterien.

Die Untersuchungen auf die antibakterielle Wirksamkeit wurden nach dem Agar-Diffusionstest und nach dem Reihen-Verdünnungstest in Anlehnung an die von P. Klein in «Bakteriologische Grundlagen der chemotherapeutischen Laboratoriumspraxis», Springer-Verlag, 1957, Seiten 53 bis 76 und 87 bis 109 beschriebenen Methodik durchgeführt.

Vergleichend untersucht wurden die bestwirksamen Verbindungen der FR-A-2 306 704

A = N-(3-Äthylaminopropyl)-erythromycylamin

B = N-(3-isopentylaminopropyl)-erythromycylamin

C = N-(3-(p-Chlor)-benzylaminopropyl)-erythromycylamin

D = N-(3-(p-Methoxy)-benzylaminopropyl)-erythromycylamin

E = N-[2-(Pyridyl-2-amino)äthyl]-erythromycylamin (vgl. S. 14, Zeile 26)

mit den folgenden Verbindungen der obigen allgemeinen Formel I

F = N-(3-(2-Dimethylamino-pyrimidin-4-yl-amino)propyl)-erythromycylamin (Beispiel 1)

G = N-(3-(2-Dimethylamino-5-methyl-pyrimidin-4-yl-amino)-propyl)-erythromycylamin (Beispiel 1f)

H = N-(3-[2-Dimethylamino-5.6.7.8-tetrahydrochinazolin-4-yl-amino]propyl)-erythromycylamin (Beispiel 1s)

I = N-(3-[6-Dimethylamino-pyrimidin-4-yl-amino]propyl)-erythromycylamin (Beispiel 1i)

bezüglich ihrer minimalen Hemmkonzentration im Reihenverdünnungstest (MIC in µg/ml), ihrer therapeutischen Wirkung nach oraler Applikation bei Streptococcus pyogenes ATCC 11775-Infektionen an der Maus, der von ihnen erzeugten Serum-, Leber- und Nierenspiegel an der Ratte und ihrer orientierenden Toxizität nach peroraler Applikation an der Maus.

1. In vitro-Versuche zur Bestimmung der minimalen Hemmkonzentration

Für die Untersuchungen wurde die Methode des Reihenverdünnungstestes im Mikrotitersystem angewandt. Die Prüfung der Substanzen auf Bakteriostase erfolgte in flüssigem Medium. Es wurde die Bakteriostasewirkung bei folgenden Konzentrationen untersucht:

80; 40; 20;10; 5; 2,5; 1,25; 0,6 und 0,3 µg/ml. Als Nährmedium diente eine Bouillon aus 5 g Pepton, 3 g Fleischextrakt, aufgefüllt mit destilliertem

Wasser auf ein Volumen von 1000 ml; ph-Wert 6,7. Das Alter der Primärkulturen betrug 24 Stunden. Die Einstellung der Keimsuspension erfolgte am Photometer (nach «Eppendorf») (Reagenzglas-Durchmesser 14 mm, Filter 546 nm) anhand der Trübung einer Bariumsulfat-Vergleichssuspension, die durch eine Bariumsulfat-Aufschwemmung erzeugt wurde, welche durch die Zugabe von 3,0 ml 1%ige Bariumchloridlösung in 97 ml 1%ige Schwefelsäure entstand. Nach der Einstellung wurden Streptococcus Aronson im Verhältnis 1:150 und die übrigen Testkeime im Verhältnis 1:1500 mit einer Kochsalzlösung weiter verdünnt.

16 mg der jeweiligen Substanz wurden in 10 ml-Messkolben eingewogen und mit dem Lösungsmittel bis zur Marke aufgefüllt. Die weitere Verdünnungsreihe wurde mit destilliertem Wasser oder dem jeweiligen Lösungsmittel hergestellt.

Die Vertiefungen der Mikrotiterplatten wurden mit 0,2 ml Nährmedium, 0,01 ml der entsprechenden Substanzverdünnung und mit einem Tropfen Keimsuspension beschickt und 18 bis 20 Stunden bei 37 °C bebrütet. Eine Lösungsmittelkontrolle wurde stets mitgeführt.

Die Ablesung wurde makroskopisch vorgenommen, wobei die jeweilige Grenzkonzentration (= niedrigste noch bakteriostatisch wirksame Konzentration) ermittelt wurde.

Als Testorganismen wurden benutzt:

Staphylococcus aureus SG 511 } grampositive Keime
Streptococcus Aronson

Escherichia coli ATCC 9637
Pseudomonas aeruginosa Hamburgensis } gramnegative Keime
Klebsiella pneumoniae ATCC 10031

Die nachfolgende Tabelle 1 enthält die dabei gefundenen Werte:

Tabelle 1

| Verbindung | MIC (μg/ml) | | | | |
|---|---|---|---|---|---|
| | Staph. aureus SG 511 | Sc. Aronson | E. coli. ATCC 9637 | PS. aerug. Hbg. | Kleb. pneumon ATCC 1003 |
| A | 1,25 | 0,31 | 20 | 40 | |
| B | 5,0 | 0,08 | 10 | 40 | |
| C | 1,25 | 0,08 | 5,0 | 80 | |
| D | 1,25 | 0,08 | 5,0 | >80 | |
| E | 0,31 | 0,31 | 10 | 40 | 20 |
| F | 0,31 | 0,08 | 1,25 | 10 | 5 |
| G | 0,31 | 0,08 | 2,5 | 20 | |
| H | 1,25 | 0,02 | 2,5 | 20 | |
| I | 0,63 | 0,02 | 5,0 | 40 | |

2. Bestimmung der Serum-, Leber- und Nierenspiegel

Die Substanzen wurden im Wasser in 0,8%iger Tylose (pH = 8,0) homogenisiert.

Die zu applizierende Dosis von 10 bzw. 100 mg/kg wurde in einem Volumen von 1 ml/100 g Ratte verabreicht.

1 Stunde vor der Probengewinnung (¼ bzw. ½ Stunde bei den beiden ersten Entnahmen) wurden je 3 Ratten mit Chloralose-Urethan-Lösung narkotisiert. Zum vorgesehenen Entnahmezeitpunkt wurden die Tiere geöffnet und folgende Proben genommen:

- 1,5 ml Blut durch Herzpunktion
- 1 g Leber
- beide Nieren.

Die Bestimmung der Spiegel erfolgte mit Hilfe des Zylinderplattentestes.

Die gefundenen Serum- und Gewebskonzentrationen (μg/ml) sind in Tabelle 2 zusammengefasst.

Tabelle 2
Spiegelwerte (Ratte, μg/ml)

| Substanz | Dosierung mg/kg | Serum 0,5$^h$ | 1$^h$ | 2$^h$ | 4$^h$ | 8$^h$ | 16$^h$ | 24$^h$ | 48$^h$ |
|---|---|---|---|---|---|---|---|---|---|
| A | 100 | 0,48 | 0,17 | 0,22 | 0,06 | 0,05 | 0,06 | 0,05 | <0,05 |
| E | 100 | 0,06 | 0,06 | 0,07 | 0,1 | 0,06 | 0,09 | 0,04 | <0,04 |
| F | 10 | 0,50 | 0,48 | 0,31 | 0,25 | 0,16 | 0,19 | 0,14 | 0,14 |

Nachweisgrenze: 0,04 μg/ml

Tabelle 2 (Fortsetzung)
Spiegelwerte (Ratte, µg/ml)

| Substanz | Dosierung mg/kg | Leber $0,5^h$ | $1^h$ | $2^h$ | $4^h$ | $8^h$ | $16^h$ | $24^h$ | $48^h$ |
|---|---|---|---|---|---|---|---|---|---|
| A | 100 | <0,4 | <0,4 | <0,04 | <0,04 | <0,4 | 1,35 | 0,89 | 0,42 |
| E | 100 | <0,35 | <0,35 | 0,4 | 0,42 | 0,51 | 0,9 | 0,45 | 0,4 |
| F | 10 | 2,04 | 1,95 | 2,10 | 2,14 | 2,28 | 2,99 | 2,31 | 2,19 |

Nachweisgrenze: 0,35 µg/ml

| Substanz | Dosierung mg/kg | Niere $0,5^h$ | $1^h$ | $2^h$ | $4^h$ | $8^h$ | $16^h$ | $24^h$ | $48^h$ |
|---|---|---|---|---|---|---|---|---|---|
| A | 100 | 0,67 | 0,45 | 0,48 | 0,41 | 0,45 | 0,48 | 0,43 | 0,41 |
| E | 100 | <0,35 | <0,35 | <0,35 | <0,35 | 0,4 | 0,5 | <0,35 | <0,35 |
| F | 10 | 2,09 | 1,55 | 1,64 | 1,69 | 1,73 | 2,31 | 1,94 | 1,77 |

Nachweisgrenze: 0,35 µg/ml

3. Therapie-Versuch

Der Nachweis der therapeutischen Wirkung wurde am Modell der Streptococcus pyogenes ATCC 11775-Infektion der Maus geführt. Dazu wurden Gruppen von 10 Mäusen (NMRI, 18–20 g, ♀) mit ca. $8\times10^2$ Keimen/Maus (in 0,2 ml) ip. infiziert und sc. 1×2 Stunden post infectionem behandelt. Für die Beurteilung der Substanzwirkung wird sowohl das Überleben der Infektion als auch die Verzögerung in der Absterbegeschwindigkeit berücksichtigt.

Tabelle 3
Sc. pyogenes ATCC 8668 Infektion

| Verbindung | $DE_{50}$ (Maus) mg/kg po. |
|---|---|
| A | >128 |
| B | >128 |
| C | >128 |
| D | >128 |
| E | >128 |
| F | 10,6 |
| G | 11,8 |
| H | 21,5 |
| I | 23,8 |

4. Toxizität

Die akute Toxizität wurde durch perorale Applikation der Verbindungen an weisse NMRI-Mäuse beiderlei Geschlechts (18–22 g) in steigenden Dosen bestimmt:

Jeweils 10 Mäusen wurde 500, 1000 und 2000 mg/kg appliziert. Die Zahl der Todesfälle wurde über einen Zeitraum von 14 Tagen registriert.

Tabelle 4

| Substanz | in der Beobachtungszeit gestorbene Tiere 500 mg/kg | 1000 mg/kg | 2000 mg/kg |
|---|---|---|---|
| A | 1 | 1 | 4 |
| B | 1 | 2 | 5 |
| C | 0 | 0 | 2 |
| D | 1 | 1 | 3 |
| E | 0 | 0 | 1 |
| F | 0 | 0 | 1 |
| G | 0 | 0 | 0 |
| H | 0 | 0 | 0 |
| I | 0 | 1 | 2 |

Für die Substanzen A und B liegt die $LD_{50}$ an der Maus bei ungefähr 2000 mg/kg po, für die Substanzen C bis H über 2000 mg/kg po.

Wie die in-vitro-Versuche zur Bestimmung der minimalen Hemmkonzentration (MIC = minimum inhibitory concentration) zeigen, sind die Substanzen F bis I den bekannten Substanzen A bis E durchschnittlich um das 2- bis 5fache sowohl in ihrer Wirkung gegen grampositive als auch gegen gramnegative Keime überlegen.

Die Substanz F zeigt gegenüber den Substanzen A und E selbst in einer geringeren Dosierung (10 mg/kg p.o. gegenüber 100 mg/kg p.o. bei den Substanzen A und E) noch signifikant erhöhte Serum- und Gewebsspiegel (die Gewebsspiegel sind meistens um das 3- bis 6fache erhöht).

Die Substanzen F bis I zeigen eine um mehr als den Faktor 10 erhöhte therapeutische Wirkung in einem Modellversuch an der Maus, verglichen mit den Substanzen A bis E.

Darüber hinaus sind die Substanzen F bis I im Vergleich zu den Substanzen A bis E etwas weniger toxisch.

Besonders gut antibakteriell noch in Konzentrationen von 0,3 bis 5,0 µg/ml gegen Staphylococcus aureus SG 511 und Streptococcus Aronson und in Konzentrationen von 5 bis 40 µg/ml gegen Escherichia coli wirken zum Beispiel die folgenden Substanzen:

N-(3-[2-Dimethylamino-pyrimidin-4-ylamino]propyl)-erythromycylamin
N-(3-[2-Dimethylamino-5-methyl-pyrimidin-4-ylamino]propyl)-erythromycylamin
N-(3-[2-Dimethylamino-6-methyl-pyrimidin-4-ylamino]propyl)-erythromycylamin
N-(3-[6-Dimethylamino-pyrimidin-4-ylamino]propyl)-erythromycylamin
N-(3-[2-Dimethylamino-6-äthyl-pyrimidin-4-ylamino]propyl)-erythromycylamin
N-(3-[2-Dimethylamino-5,6,7,8-tetrahydrochinazolin-4-ylamino]propyl)-erythromycylamin
N-(3-[4-Dimethylamino-pyrimidin-2-ylamino]propyl)-erythromycylamin
N-(3-[4-Phenyläthylamino-pyrimidin-2-ylamino]propyl)-erythromycylamin
N-(3-[2-Dimethylamino-5-phenyl-pyrimidin-4-ylamino]propyl)-erythromycylamin
N-(3-[2-Amino-5-methyl-pyrimidin-4-ylamino]propyl)-erythromycylamin
N-(3-[4-Dimethylamino-chinazolin-2-ylamino]propyl)-erythromycylamin
N-(3-[4-Dimethylamino-thieno[3,2-d]pyrimidin-2-ylamino]propyl)-erythromycylamin
N-(3-[4-Phenoxyäthylamino-pyrimidin-2-ylamino]propyl)-erythromycylamin

Die akute Toxizität, bestimmt an der Maus, liegt bei allen vorstehend genannten Verbindungen bei oraler und subkutaner Applikation bei $LD_{50}$-Werten über 1,5 g/kg.

Die nachfolgenden Beispiele sollen die Erfindung näher erläutern:

Beispiel 1

N-(3-[2-Dimethylamino-pyrimidin-4-ylamino]propyl)-erythromycylamin

5,8 g (0,0073 Mol) N-(3-Aminopropyl)-erythromycylamin, 1,18 g (0,0075 Mol) 4-Chlor-2-dimethylamino-pyrimidin und 0,7 g (0,007 Mol) Triäthylamin werden in 50 ml absolutem Äthanol gelöst und 4 Stunden im geschlossenen Gefäss auf 140 °C erhitzt.

Nach dem Abkühlen wird das Lösungsmittel im Vakuum abdestilliert, der feste Rückstand in Methylenchlorid gelöst und diese Lösung 3mal mit Wasser extrahiert. Die organische Phase wird über Natriumsulfat getrocknet, das Lösungsmittel im Vakuum abdestilliert, und der feste Rückstand durch Säulenchromatographie gereinigt (Adsorbens: Aluminiumoxid basisch (Firma Woelm), Eluens: Chloroform/Methanol (60+0,8)).

Nach Abdestillieren des Elutionsmittels erhält man eine feinkristalline, weisse Substanz.

Ausbeute: 3,1 g (47 % der Theorie)
F.: 101 °C (Zers.)
$C_{46}H_{84}N_6O_{12}$ (913.23)

| | | | |
|---|---|---|---|
| Ber.: | C 60.50 | H 9.27 | N 9.20 |
| Gef.: | 60.20 | 9.34 | 9.40 |

Analog wurden die folgenden Verbindungen synthetisiert (in den Beispielen werden die Ausgangssubstanzen N-(3-Aminopropyl)-erythromycylamin als A und N-(2-Aminoäthyl)-erythromycylamin als B bezeichnet):

a) N-(3-[2-Diäthylamino-pyrimidin-4-ylamino]propyl)-erythromycylamin
durch Umsetzung von A mit 4-Chlor-2-diäthylamino-pyrimidin.
Schmelzpunkt: 99 °C (Zers.)

b) N-(3-[2-Dimethylamino-6-methyl-pyrimidin-4-ylamino]propyl)-erythromycylamin
durch Umsetzung von A mit 4-Chlor-2-dimethylamino-6-methyl-pyrimidin.
Schmelzpunkt: 109 °C (Zers.)

c) N-(3-[6-Äthyl-2-dimethylamino-pyrimidin-4-ylamino]propyl)-erythromycylamin
durch Umsetzung von A mit 6-Äthyl-4-chlor-2-dimethylamino-pyrimidin.
Schmelzpunkt: 91 °C (Zers.)

d) N-(3-[2-Dimethylamino-6-isopropyl-pyrimidin-4-ylamino]propyl)-erythromycylamin
durch Umsetzung von A mit 4-Chlor-2-dimethylamino-6-isopropyl-pyrimidin.
Schmelzpunkt: 118 °C (Zers.)

e) N-(3-[2-Dimethylamino-6-phenyl-pyrimidin-4-ylamino]propyl)-erythromycylamin
durch Umsetzung von A mit 4-Chlor-2-dimethylamino-6-phenyl-pyrimidin.
Schmelzpunkt: 123 °C (Zers.)

f) N-(3-[2-Dimethylamino-5-methyl-pyrimidin-4-ylamino]propyl)-erythromycylamin
durch Umsetzung von A mit 4-Chlor-2-dimethylamino-5-methyl-pyrimidin.
Schmelzpunkt: 114 °C (Zers.)

g) N-(3-[2-Dimethylamino-5-phenyl-pyrimidin-4-ylamino]propyl)-erythromycylamin
durch Umsetzung von A mit 4-Chlor-2-dimethylamino-5-phenyl-pyrimidin.
Schmelzpunkt: 93 °C (Zers.)

h) N-(3-[5.6-Dimethyl-2-dimethylamino-pyrimidin-4-ylamino]propyl)-erythromycylamin
durch Umsetzung von A mit 4-Chlor-5.6-dimethyl-2-dimethylamino-pyrimidin.
Schmelzpunkt: 103 °C (Zers.)

i) N-(3-[6-Dimethylamino-pyrimidin-4-ylamino]propyl)-erythromycylamin
durch Umsetzung von A mit 4-Chlor-6-dimethylamino-pyrimidin.
Schmelzpunkt: 100 °C (Zers.)

k) N-(3-[2-Amino-6-methyl-pyrimidin-4-ylamino]propyl)-erythromycylamin
durch Umsetzung von A mit 2-Amino-4-chlor-6-methyl-pyrimidin.
Schmelzpunkt: 121 °C (Zers.)

l) N-(3-[2-Amino-5-methyl-pyrimidin-4-ylamino]propyl)-erythromycylamin
durch Umsetzung von A mit 2-Amino-4-chlor-5-methyl-pyrimidin.
Schmelzpunkt: 119 °C (Zers.)

m) N-(3-[4-Dimethylamino-pyrimidin-2-ylamino]propyl)-erythromycylamin
durch Umsetzung von A mit 2-Chlor-4-dimethylamino-pyrimidin.
Schmelzpunkt: 108 °C (Zers.)

n) N-(3-[4-[2-Phenyl]äthylamino-pyrimidin-2-ylamino]propyl)-erythromycylamin
durch Umsetzung von A mit 2-Chlor-4-(2-phenyl)-äthylamino-pyrimidin.
Schmelzpunkt: 94 °C (Zers.)

o) N-(3-[4-[1-Phenyl]äthylamino-pyrimidin-2-ylamino]propyl)-erythromycylamin
durch Umsetzung von A mit 2-Chlor-4-(1-phenyl)-äthylamino-pyrimidin.
Schmelzpunkt: 97 °C (Zers.)

p) N-(3-[4-[2-Phenoxy]äthylamino-pyrimidin-2-ylamino]propyl)-erythromycylamin
durch Umsetzung von A mit 2-Chlor-4-(2-phenoxy)-äthylamino-pyrimidin.
Schmelzpunkt: 73 °C (Zers.)

q) N-(3-[Pyrimidin-2-ylamino]propyl)-erythromycylamin
durch Umsetzung von A mit 2-Chlor-pyrimidin.
Schmelzpunkt: 109 °C (Zers.)

r) N-(2-[4-Dimethylamino-pryimidin-2-ylamino]äthyl)-erythromycylamin
durch Umsetzung von B mit 2-Chlor-4-dimethylamino-pyrimidin.
Schmelzpunkt: 103 °C (Zers.)

s) N-(3-[2-Dimethylamino-5.6.7.8-tetrahydrochinazolin-4-ylamino]propyl)-erythromycylamin
durch Umsetzung von A mit 2-Dimethylamino-4-chlor-5.6.7.8-tetrahydro-chinazolin.
Schmelzpunkt: 116 °C (Zers.)

t) N-(3-[4-Dimethylamino-chinazolin-2-ylamino]propyl)-erythromycylamin
durch Umsetzung von A mit 2-Chlor-4-dimethylamino-chinazolin.
Schmelzpunkt: 102 °C (Zers.)

u) N-(3-[4-Dimethylamino-thieno[3.2-d]pyrimidin-2-ylamino]propyl)-erythromycylamin
durch Umsetzung von A mit 2-Chlor-4-dimethylamino-thieno[3.2-d]pyrimidin
Schmelzpunkt: 120 °C (Zers.)

Beispiel 2

N-(3-[2-Dimethylamino-6-methyl-pyrimidin-4-ylamino]propyl)-erythromycylamin

7,92 g (0,01 Mol) N-(3-aminopropyl)-erythromycylamin und 2,15 g (0,01 Mol) 2-Dimethylamino-6-methyl-4-methylsulfonyl-pyrimidin werden in 50 ml Dimethylsulfoxid gelöst und 5 Stunden auf 100 °C erhitzt.

Nach dem Abkühlen wird das Lösungsmittel im Hochvakuum abdestilliert, der Rückstand in Methylenchlorid gelöst und mit Wasser extrahiert. Man trocknet die organische Phase über Natriumsulfat, zieht das Lösungsmittel ab und reinigt den verbleibenden Rückstand säulenchromatographisch.

(Adsorbens: Aluminiumoxid basisch (Firma Woelm), Eluens: Chloroform/Methanol (30+0,5)).

Die gewünschte Verbindung wird als weisses, kristallines Pulver erhalten.

Ausbeute: 4,7 g (51% der Theorie)
F.: 109 °C (Zers.)

$C_{47}H_{86}N_6O_{12}$ (927.25)

| | | | | | | |
|---|---|---|---|---|---|---|
| Ber.: | C | 60.88 | H | 9.35 | N | 9.06 |
| Gef.: | | 60.90 | | 9.38 | | 9.01 |

Die Verbindungen der allgemeinen Formel I lassen sich in an sich bekannter Weise in die üblichen pharmazeutischen Zubereitungsformen, zum Beispiel in Lösungen, Suppositorien, Tabletten, einarbeiten. Die Einzeldosis beträgt für Erwachsene bei peroraler Applikation 50 bis 500 mg, die bevorzugte Einzeldosis 100 bis 250 mg, die Tagesdosis 0,5 bis 4 g, die bevorzugte Tagesdosis 1 bis 2 g.

Die nachfolgenden Beispiele sollen die Herstellung einiger pharmazeutischer Zubereitungen verdeutlichen:

Beispiel I

Tabletten mit 100 mg N-(3-[2-Dimethylamino-pyrimidin-4-ylamino]propyl)-erythromycylamin

Zusammensetzung:
1 Tablette enthält:

| | |
|---|---|
| Wirkstoff | 100,0 mg |
| Milchzucker | 63,0 mg |
| Kartoffelstärke | 50,0 mg |
| Polyvinylpyrrolidon | 5,0 mg |
| Magnesiumstearat | 2,0 mg |
| | 220,0 mg |

Herstellungsverfahren:

Die Mischung der Wirksubstanz mit Milchzucker und Kartoffelstärke wird mit einer 10%igen wässrigen Lösung des Polyvinylpyrrolidons befeuchtet, durch ein Sieb der Maschenweite 1,5 mm granuliert, bei 45 °C getrocknet und nochmals durch obiges Sieb gerieben. Das so erhaltene Granulat wird mit Magnesiumstearat gemischt und zu Tabletten verpresst.

Tablettengewicht: 220 mg
Stempel: 9 mm flach, mit Teilkerbe

Beispiel II

Dragées mit 100 mg N-(3-[2-Dimethylamino-pyrimidin-4-ylamino]propyl)-erythromycylamin

Zusammensetzung:
1 Dragéekern enthält:

| | |
|---|---|
| Wirkstoff | 100,0 mg |
| Milchzucker | 30,0 mg |
| Maisstärke | 30,0 mg |
| Gelatine | 3,0 mg |
| Cellulose, mikrokristallin | 6,0 mg |
| Magnesiumstearat | 1,0 mg |
| | 170,0 mg |

Herstellungsverfahren:

Die Mischung der Wirksubstanz mit Milchzucker und Maisstärke wird mit einer 12%igen wässrigen Lösung der Gelatine befeuchtet, durch ein Sieb der Maschenweite 1,5 mm granuliert, bei 45 °C getrocknet und nochmals durch ein Sieb der Maschenweite 1,0 mm gerieben. Das so erhaltene Granulat wird mit Cellulose und Magnesiumstearat gemischt und zu Dragéekernen verpresst.

Kerngewicht: 170 mg
Stempel: 7 mm, gewölbt.

Die so erhaltenen Dragéekerne werden nach bekannten Verfahren mit einer Hülle überzogen,

die im wesentlichen aus Zucker und Talkum besteht. Die fertigen Dragées werden mit Hilfe von Bienenwachs poliert.
Dragéegewicht: 210 mg.

**Patentansprüche**

1. 9-(ω-Heteroarylamino-alkylamino)-erythromycine der allgemeinen Formel

$$R\text{-}NH\text{-}(CH_2)_n\text{-}NH\text{-}E \qquad (I)$$

in der
E die Erythromycylgruppe +

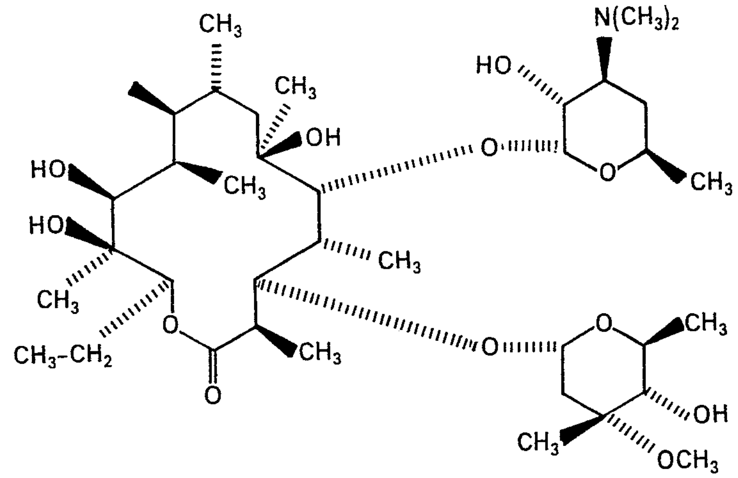

+ (Strukturformel nach E.H. Massey et al., J. Med. Chem. 17, 105 [1974])
n die Zahl 2 oder 3 und
R die 2-Pyrimidinylgruppe der allgemeinen Formel

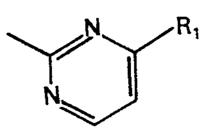

bedeuten, in der $R_1$ ein Wasserstoff, eine Dialkylaminogruppe mit 2 bis 6 Kohlenstoffatomen, eine Phenoxyalkylaminogruppe mit 1–3 C-Atomen im Alkylenteil oder eine Phenylalkylaminogruppe mit 1 bis 3 Kohlenstoffatomen im Alkylenrest, darstellt,
oder in der
R die 4-Pyrimidinylgruppe der allgemeinen Formel

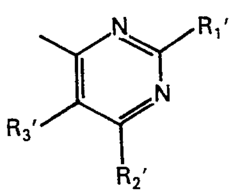

in der
$R_1'$ ein Wasserstoffatom, die freie Aminogruppe oder eine Dialkylaminogruppe mit 2 bis 6 Kohlenstoffatomen,
$R_2'$ ein Wasserstoffatom, einen geradkettigen oder verzweigten Alkylrest mit 1 bis 3 Kohlenstoffatomen, einen Phenylrest, oder eine Dialkylaminogruppe mit insgesamt 2 bis 6 Kohlenstoffatomen und
$R_3'$ ein Wasserstoffatom, einen geradkettigen oder verzweigten Alkylrest mit 1 bis 3 Kohlenstoffatomen, oder einen Phenylrest darstellen oder
R die 2-Chinazolinylgruppe der allgemeinen Formel

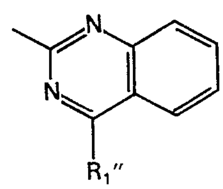

die 4-(5,6,7,8-Tetrahydro)-chinazolinylgruppe der allgemeinen Formel

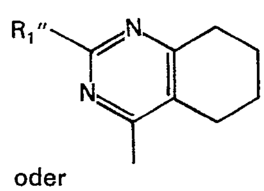

oder
die 2-Thieno[3,2-d]pyrimidinylgruppe der allgemeinen Formel

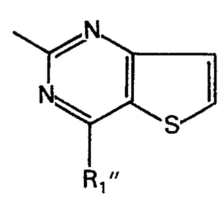

bedeutet, wobei in den obigen Formeln $R_1''$ ein Wasserstoffatom, oder eine Dialkylaminogruppe mit 2 bis 6 Kohlenstoffatomen bedeutet und deren pharmakologisch verträgliche Salze mit anorganischen oder organischen Säuren.

2. N-[3-(2-Dimethylamino-pyrimidin-4-ylamino]propyl)-erythromycylamin und dessen pharmakologisch verträgliche Salze mit anorganischen oder organischen Säuren.

3. Verfahren zur Herstellung von 9-(ω-Heteroarylamino-alkylamino)-erythromycinen und deren Salzen mit anorganischen oder organischen Säuren gemäss Anspruch 1, dadurch gekennzeichnet, dass ein Aminoalkylamino-erythromycin der allgemeinen Formel II,

$H_2N-(CH_2)_n-NH-E$ (II)

in der
n und E wie oben definiert sind, mit einer Verbindung der allgemeinen Formel III,

R-Z (III)

in der
R wie oben definiert ist und Z eine leaving group bedeutet, in einem polaren Lösungsmittel bei Temperaturen zwischen 20 und 150 °C umgesetzt wird und die so erhaltenen Verbindungen gewünschtenfalls in ihre Salze mit anorganischen oder organischen Säuren überführt werden.

4. Verfahren gemäss Anspruch 3, dadurch gekennzeichnet, dass eine Verbindung der allgemeinen Formel III, in der Z ein Halogenatom ist, mit einer Verbindung der allgemeinen Formel II in Gegenwart eines halogenwasserstoffbindenden Mittels umgesetzt wird.

5. Pharmazeutische Zubereitung, enthaltend eine oder mehrere Verbindungen nach Anspruch 1 und übliche Träger- und Zusatzstoffe.

**Patent Claims**

1. 9-(ω-heteroarylamino-alkylamino)-erythromycins of general formula

$R-NH-(CH_2)_n-NH-E$ (I)

in which
E represents the erythromycyl group*

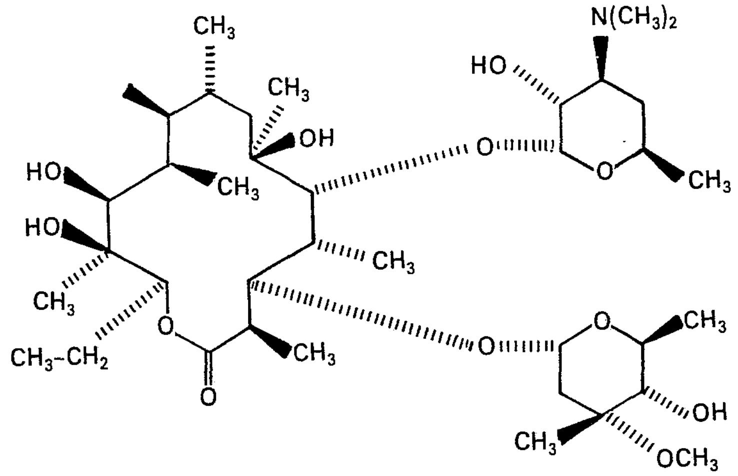

* (Structural formula according to E.H. Massey et al., J. Med. Chem. 17, 105 [1974]).
n represents the integer 2 or 3 and
R represents the 2-pyrimidinyl group of general formula

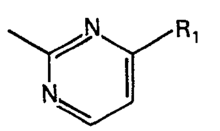

in which $R_1$ represents a hydrogen atom, a dialkylamino group with 2 to 6 carbon atoms, a phenoxyalkylamino group with 1 to 3 carbon atoms in the alkylene part or a phenylalkylamino group with 1 to 3 carbon atoms in the alkylene radical, or in which
R represents the 4-pyrimidinyl group of general formula

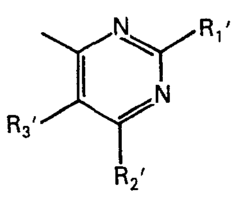

in which
$R_1'$ represents a hydrogen atom, the free amino group or a dialkylamino group with 2 to 6 carbon atoms,
$R_2'$ represents a hydrogen atom, a straight-chained or branched alkyl radical with 1 to 3 carbon atoms, a phenyl radical or a dialkylamino group with altogether 2 to 6 carbon atoms and
$R_3'$ represents a hydrogen atom, a straight-chained or branched alkyl radical with 1 to 3 carbon atoms or a phenyl radical or
R represents the 2-quinazolinyl group of general formula

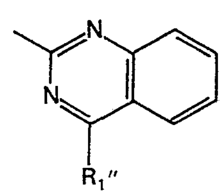

the 4-(5,6,7,8-tetrahydro)-quinazolinyl group of general formula

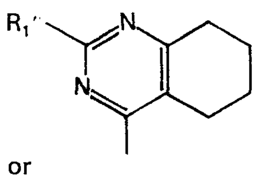

or

the 2-thieno[3,2-d]pyrimidinyl group of general formula

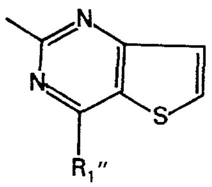

where in the above formulas $R_1''$ represents a hydrogen atom or a dialkylamino group with 2 to 6 carbon atoms, and their pharmacologically compatible salts with inorganic or organic acids.

2. N-[3-(2-Dimethylamino-pyrimidin-4-ylamino)-propyl]-erythromcyclamine and its phramacologically compatible salts with inorganic or organic acids.

3. Process for the preparation of 9-(ω-heteroarylamino-alkylamino)-erythromycins and their salts with inorganic or organic acids according to claim 1, characterised in that anaminoalkylamino-erythromcycin of general formula II

$$H_2N-(CH_2)_n-NH-E \quad (II)$$

in which
n and E are as defined above, is reacted with a compound of general formula III

$$R-Z \quad (III)$$

in which
R is as defined above and Z represents a leaving group, in a polar solvent at temperatures of between 20 and 150 °C and the compounds thus obtained are, if desired, converted into their salts with inorganic or organic acids.

4. Process according to claim 3, characterised in that a compound of general formula III in which Z is a halogen atom is reacted with a compound of general formula II in the presence of a hydrogen-halide-binding agent.

5. Pharmaceutical preparation containing one or more compounds according to claim 1 and conventional carriers and additives.

**Revendications**

1. 9-(ω-hétéroarylamino-alcoylamino)-érythromycines de formule générale

$$R-NH-(CH_2)_n-NH-E \quad (I)$$

dans laquelle
E représente le groupe érythromycyle*

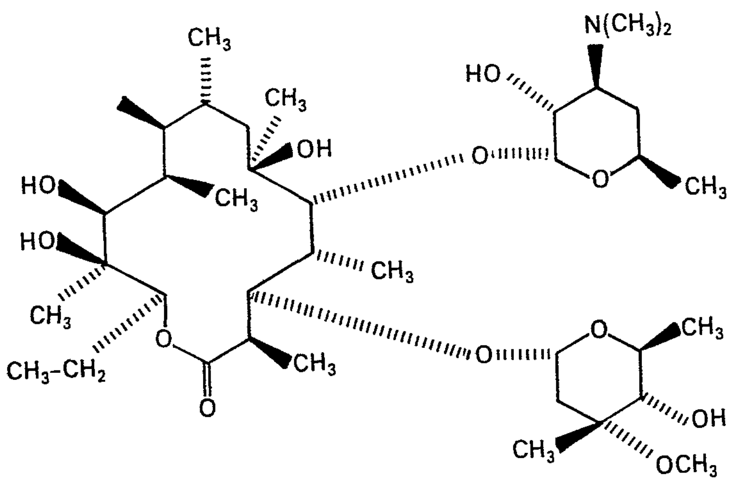

* (formule de constitution selon E.H. Massey et Coll., J. Med. Chem. 17, 105 [1974])

n représente le nombre 2 ou 3 et
R représente le groupe 2-pyrimidinyle de formule générale

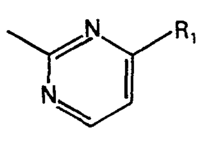

dans laquelle $R_1$ représente un atome d'hydrogène, un groupe dialcoylamino avec 2 à 6 atomes de carbone, un groupe phénoxyalcoylamino avec 1-3 atomes de carbone dans la partie alcoylène ou un groupe phénylalcoylamino avec 1 à 3 atomes de carbone dans le radical alcoylène,
ou dans laquelle
R représente le groupe 4-pyrimidinyle de formule générale

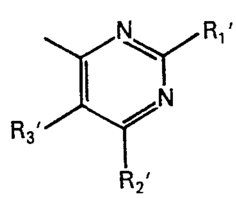

dans laquelle

$R_1'$ représente un atome d'hydrogène, le groupe amino libre ou un groupe dialcoylamino avec 2 à 6 atomes de carbone,

$R_2'$ représente un atome d'hydrogène, un radical alcoyle rectiligne ou ramifié avec 1 à 3 atomes de carbone, un radical phényle ou un groupe dialcoylamino avec en tout 2 à 6 atomes de carbone et

$R_3'$ représente un atome d'hydrogène, un radical alcoyle rectiligne ou ramifié avec 1 à 3 atomes de carbone, ou un radical phényle ou

R représente le groupe 2-quinazolinyle de formule générale

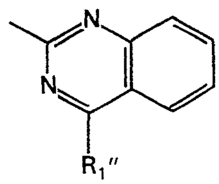

le groupe 4-(5,6,7,8-tétrahydro)-quinazolinyle de formule générale

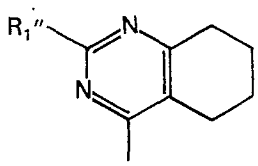

ou

le groupe 2-thiéno[3,2-d]pyrimidinyle de formule générale

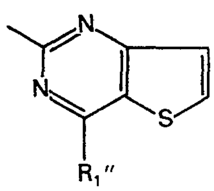

dans les susdites formules $R_1''$ représentant un atome d'hydrogène ou un groupe dialcoylamino avec 2 à 6 atomes de carbone, et leurs sels pharmacologiquement acceptables avec des acides minéraux ou organiques.

2. La N-[3-(2-diméthylamino-pyrimidine-4-yl-amino)-propyl]-érythromycylamine et ses sels pharmacologiquement acceptables avec des acides minéraux ou organiques.

3. Procédé pour la préparation des 9-(ω-hétéroarylamino-alcoylamino)-érythromycines et de leurs sels avec des acides minéraux ou organiques selon la revendication 1, caractérisé en ce que

l'on fait réagir une aminoalcoylamino-érythromycine de formule générale I

$$H_2N-(CH_2)_nNH-E \qquad (II)$$

dans laquelle

n et E sont définis comme plus haut, avec un composé de formule générale III,

$$R-Z \qquad (III)$$

dans laquelle

R est défini comme plus haut et Z représente un groupe partant, dans un solvant polaire à des températures comprises entre 20 et 150 °C et en ce que l'on transforme, si on le désire, les composés ainsi obtenus en leurs sels avec des acides minéraux ou organiques.

4. Procédé selon la revendication 3, caractérisé en ce que l'on fait réagir un composé de formule générale III, dans laquelle Z est un atome d'halogène, avec un composé de formule générale II en présence d'un agent fixant les halogénures d'hydrogène.

5. Préparation pharmaceutique renfermant un ou plusieurs composés selon la revendication 1 et des excipients et additifs habituels.